# EUROPEAN PATENT SPECIFICATION

(11) **EP 3 843 666 B1**
(45) Date of publication and mention of the grant of the patent: **22.01.2025**
(21) Application number: 20701349.1
(22) Date of filing: 27.01.2020
(51) Int. Cl.: A61F 2/24, A61F 2/852, A61F 2/95

(54) **THREE PART STENT SECOND GENERATION**
DREITEILIGER STENT DER ZWEITEN GENERATION
SECONDE GÉNÉRATION D'ENDOPROTHÈSE VASCULAIRE EN TROIS PARTIES

(30) Priority: 28.01.2019 EP 19153905
(43) Date of publication of application: 07.07.2021
(60) Divisional application: 23209649.5
(73) Proprietor: Tricares SAS, 75001 Paris (FR)
(72) Inventor: RAHMIG, Georg, 75175 Pforzheim (DE); STRAUBINGER, Helmut, 85609 Aschheim (DE)
(74) Representative: Hoefer & Partner Patentanwälte mbB
(86) International application number: PCT/EP2020/051955
(87) International publication number: WO 2020/157018

(56) References cited:
- EP-A1- 3 202 371
- WO-A1-2018/217338
- US-A1- 2011 264 207
- US-A1- 2018 055 629
- US-A1- 2018 338 832

## Description

The present invention relates to a novel stent and a replacement heart valve prosthesis.

In the last decades minimally invasive techniques and catheter-based implantation techniques have advanced and are now feasible in many medical fields.

In a number of medical fields it is now possible to treat patients by catheter-based techniques allowing for the treatment of such patients who could otherwise not be adequately taken care of due to their physical condition and the risks connected with surgery. Such catheter-based techniques apply to delivery systems, e.g. a catheter and/or introducer sheath, for implanting the medical device to a desired target site via different access routes into a patient's body.

In particular, in recent years the treatment of heart valve diseases and deficiencies has become more and more successful. Examples are trans-apical, trans-jugular and trans-femoral procedures for heart valve replacement therapies, e.g. aortic or mitral heart valve treatments. Such example is disclosed in US 2018/338832A1.

In many cases a stent-based prosthesis with a tissue based replacement valve is used and implanted to replace the native heart valve using a catheter delivery system.

The replacement heart valve prosthesis has to be crimped and loaded onto the catheter. The correct positioning of a replacement heart valve prosthesis, durability and good compliance with the target site and the biology of the target site are important aspects of such a prosthesis.

Hence there exists a need for a replacement heart valve prosthesis which can be correctly positioned, exhibits good durability features and has as little impact on or interference with the target site as possible and which remains long term correctly positioned. In addition, a replacement heart valve prosthesis should exhibit good sealing characteristics to avoid e.g. paravalvular leakage.

In particular in soft endogenous tissue like the mitral or tricuspid heart valve, it is difficult to properly position a replacement heart valve prosthesis and provide sufficient fixation properties as well as to avoid unnecessary interference with the endogenous valve environment.

More particularly, it is a problem to durably position the replacement heart valve, e.g. in tricuspid or mitral replacement heart valve technology wherein the diameter of the valve is large and the annulus and atrial and ventricular tissue surrounding the heart valve is either sensitive or anatomically challenging for precise positioning and fixation.

Accordingly, it is one object of the current disclosure to provide a replacement heart valve prosthesis with sufficient positioning and fixation features or/and exhibiting reduced interference characteristics at the target site, or at least to provide a prosthesis wherein the disadvantages of the prior art are essentially avoided or which show reduced disadvantages vis-à-vis the prior art.

### Brief Summary of the Disclosure

In one aspect the disclosure relates to a stent or replacement heart valve prosthesis exhibiting advantageous positioning characteristics, or/and good durability characteristics or/and reduced interference characteristics.

In another aspect the disclosure relates to a stent or replacement heart valve prosthesis characterized by an inner stent combined with an outer stent wherein fixation means are connected to or form part of the inner stent.

In another aspect the disclosure relates to a replacement heart valve prosthesis comprising an inner stent and an outer stent wherein fixation means are connected to or form part of the inner stent, and at least one sealing means connected to the inner and/or outer stent and a valve connected to the inner stent.

In another aspect the disclosure relates to method for implantation or positioning a replacement heart valve prosthesis in a native tricuspid or mitral heart valve wherein the prosthesis is delivered by a catheter-based delivery system to the target site.

In another aspect the disclosure relates to a method of replacement of a malfunctioning endogenous heart valve or the implantation of a replacement heart valve prosthesis in a person experiencing impaired heart valve function.

### Brief Description of the Drawings

Various embodiments of the disclosure are exemplified by the Figures wherein:
Fig. 1a illustrates a two-part stent comprising inner and outer stent (2, 1) wherein the inner stent (2) is connected by connecting means (4) with fixation means comprising connecting arms (7), connecting arches (12) and anchoring loops (6).
Fig. 1b illustrates areas 9, 10, 11 of a replacement heart valve prosthesis according to the disclosure.
Fig. 2a, 2b, 2c depict a replacement heart valve prosthesis according to the disclosure from different angles.
Fig. 3a, 3b, 3c depict an outer stent (1) according to the disclosure from different angles.
Fig. 4a to 7 show different embodiments of fixation means (18) according to the disclosure.
Fig. 8a, 8b, 8c show different embodiments of radial force wires (17) according to the disclosure.
Fig. 9 illustrates the different areas (9), (10), (11) of a replacement heart valve prosthesis according to the disclosure implanted into a native tricuspid heart valve.
Fig. 10 to 13 show a replacement heart valve prosthesis according to the disclosure loaded onto a catheter delivery system into a delivery capsule.
Fig. 14 to 16 is a sequence illustrating the deployment of a replacement heart valve prosthesis according to the disclosure at a target site in a patient heart (tricuspid).
Fig. 17 to 19 are examples of sealing means (24, 24a, 24b, 24c) in a replacement heart valve prosthesis according to the disclosure.

### Detailed Description

In the following certain terms of the disclosure will be defined. Otherwise technical terms in the context of the disclosure shall be understood as by the applicable skilled person.

The term "prosthesis" or "medical device" or "implant" in the sense of the disclosure is to be understood as any medical device that can be delivered in a minimally invasive fashion or by way of a catheter based procedure. The terms can be used interchangeably. A prosthesis in the sense of the disclosure can be e.g. a stent or stent-based prosthesis or stent-based replacement heart valve prosthesis like an aortic replacement heart valve, a mitral replacement heart valve or a tricuspid replacement heart valve.

The term "catheter" or "delivery device" in the sense of the disclosure is to be understood as the device used to deploy a prosthesis in a patient at a determined site, e.g. to replace a heart valve like a native aortic heart valve, mitral heart valve or tricuspid heart valve.

A "mesh stent" or "braided mesh stent" or "braided stent" in the sense of the disclosure is a stent composed of wires in contrast to e.g. a laser cut nitinol tube.

A "cut stent" or "laser cut stent" in the sense of the disclosure is a stent which is laser cut from e.g. a nitinol tube.

A "stent area" or "stent areas" in the sense of the disclosure is a defined area of the outer stent, mesh stent or the replacement heart valve prosthesis and in particular it is a longitudinal section or an outer section defined as proximal, middle or distal area or atrial, annular or ventricular.

A "proximal area", "middle area", "distal area" in the sense of the disclosure denotes areas of the stent or prosthesis in relation to the operator performing implantation by use of a catheter wherein proximal is close to the operator and distal is away from the operator. "Middle area" denotes in a stent or prosthesis in the sense of the disclosure is the area between the distal and proximal area. The "proximal area" can also be denoted inflow end or inflow area and the "distal area" can also be denoted outflow end or outflow area with regards to the natural blood flow in situ, i.e. in vivo, in an individual (person or patient); proximal can also be denoted atrial, middle can be denoted annular and distal can be denoted ventricular.

An "annulus area" or "annular area" in the sense of the disclosure is either the respective area of an endogenous heart valve or it defines the respective area in the replacement heart valve or stent which is to be positioned at the implantation site and it meant to align with the endogenous annulus.

A "sub-annular area" in the sense of the disclosure is the area of the prosthesis which is in distal direction (or in inflow direction or in ventricular direction) of the annulus of the endogenous heart valve. The prosthesis may cover the "sub-annular area" with the U or V grove area and the distal area.

A "groove" in the sense of the disclosure describes an area of the stent or of the prosthesis exhibiting a smaller diameter than other areas and wherein distally and proximally of said groove other areas of the stent or prosthesis having a larger diameter are in neighborhood to said groove; said groove can have a V or U shape or combinations thereof or any other bended and useful geometries or it can be characterized by just a smaller diameter as compared to the atrial and ventricular stent areas.

A "multi-part stent" in the sense of the disclosure can refer to a "two-part stent" or a "three-part stent" wherein the inner stent is connected to a fixation means or both form an integral part and an outer stent is connected with said former parts by way of useful means. The connecting means of the inner stent and fixation means can be positioned at the atrial end or area of the inner stent. The fixation means can consist of or comprise at least two connecting means, at least two connecting arches and a respective number of anchoring loops wherein the anchoring loops can advantageously be positioned essentially in the sub-annulus area or essentially in the ventricle and thus help to preventing or limiting longitudinal movement in outflow direction of the stent or prosthesis.

A "target site" in the sense of the disclosure is the location or place where the replacement heart valve prosthesis is to be implanted and where a dysfunction or malfunction shall be treated, e.g. at the annulus of a tricuspid or mitral heart valve.

A "connection" of the stents in the sense of the disclosure is the way of fixation of two stents to each other by way of suturing, by way of a clipping or clicking mechanism or any other useful manner or connecting means to attach the stents or stent and fixation means to each other.

A "connection means" or "connecting means" in the sense of the disclosure is a mechanical or physical connection of two parts of a stent or laser cut stent. The connecting means can be by e.g. welding, gluing or any other known procedure or process or means. A connecting means can also be an attachment or clipping means which exhibits a special design and geometry for releasable or non-releasable connection.

"Fixation means" or "anchoring means" in the sense of the disclosure is a component connected with the inner stent and providing essentially for anchoring the prosthesis at the target site, optionally in cooperation with additional means. In a particular aspect a fixation means is composed of or comprises an anchoring loop, an anchoring arm, a connecting arch and an inner stent anchor, preferably a connecting means connects the inner stent anchor of the fixation means with the inner stent.

An "anchoring loop" in the sense of the disclosure is a part of a stent useful in fixation of a stent or prosthesis and which aids in avoiding movement of the stent or prosthesis at the target site. In general an anchoring loop in the sense of the disclosure is a means useful for an improved fixation of a stent or prosthesis wherein a loop is fixed to or connected with or forms part of or is an integral part of the inner stent. The "loop" or "loops" in the sense of the disclosure can have different shapes like round, square etc. and are located in a defined area in a defined pattern. A "loop" in the sense of the disclosure will exhibit a defined angle with regard to the inner stent surface and it may be designed that it may stretch out straight or flip over when the stent or prosthesis is retrieved into the catheter after an initial and possibly partial deployment.

An "angle structure" or "angle" in the sense of the disclosure is an angle between two accessory lines drawn at a certain area or stent layer in order to define a certain geometry of said stent part or arch or layer with regard to other stent structures like the inner stent.

A "radial force" in the sense of the disclosure is the force exhibited by a stent or prosthesis in radial outward direction, more particularly the force exhibited by the outer stent of the prosthesis which may be a mesh or a laser cut stent, e.g. a nitinol stent. The radial force depends on the particular mesh or cut stent design and relates to the material density, e.g. the density of wires per square area in a mesh stent, or the number of cells and size of said cells circumferentially in a certain laser cut stent level or area, e.g. the proximal/atrial, middle/annular or distal/ventricular area. The radial force in a replacement heart valve prosthesis according to the disclosure will be chosen for the outer stent or the combination of the inner and outer stent and fixation means in a magnitude to provide for good contact with the surrounding tissue and to support the fixation functionality of the stent or prosthesis and optionally also as little as possible in order to avoid an interference with the endogenous environment and biology of the target site. Thus it will be chosen in a magnitude in order to avoid interference with the implantation site and endogenous tissue and function. The radial force may be supported for its fixation function by other means, e.g. loops for fixation.

The "target area" in the sense of the disclosure is the three-dimensional space surrounding or being within the native organ like a native heart valve which can be e.g. a tricuspid or mitral heart valve.

An "atraumatic design" of the loops in the sense of the disclosure is wherein the loops or other means or parts of a stent or a prosthesis are designed to avoid any or essentially any damage of the surrounding tissue or tissue in contact with said parts or at least parts manufactured in a manner to minimize damaging or/and injuring the tissue which they contact.

"Compliance" of the stent or replacement heart valve prosthesis, e.g. comprising an inner laser cut stent within an outer mesh stent, or a laser cut inner stent within a laser cut outer stent, in the sense of the disclosure relates to a positive interference with the target tissue. "Compliance" relates to a design which exhibits good geometry adaptation of the stent or prosthesis to the implantation site and wherein the stent or prosthesis exhibits advantageous fixation characteristics, good functionality as concerns valve function and at the same a minimal interference with the endogenous heart structures and heart function.

In one aspect of the disclosure, a problem underlying the application is solved by a multiple part stent or prosthesis wherein fixation means for anchoring the stent or prosthesis are directly connected with an inner stent and wherein an outer stent is essentially touching or aligned with the surrounding endogenous tissue of the target site upon implantation.

In one aspect of the disclosure, a problem underlying the application is solved by a three part stent or prosthesis wherein the outer stent is connected with fixation means and the fixation means are connected with the inner stent while the essential fixation (anchoring) functionality at the target site is performed by the fixation means and the essential sealing functionality is performed by the outer stent and the sealing means connected therewith.

Hence in one aspect the invention provides for advantageous anchoring characteristics and/or sealing characteristics by way of connecting the anchoring means with the inner stent. It could surprisingly be found that the connection of the anchoring means to the inner stent provides for an advantageous radial force in relation to the anchoring of the stent or prosthesis at the target site. Another aspect is the advantageous force distribution due to the connection of the anchoring means with the inner stent. Another aspect is the advantage of a connection of the anchoring means only at one end of the inner stent which may lead to a positive radial force for anchoring and at the same time a decoupling of the tissue movement during heart beating. Another aspect is the advantage that the anchoring means is less coupled to the outer stent and/or that the inner and outer stents are solely coupled by a partial connection of the outer stent with the anchoring means. This may support an uncoupling of the inner and outer stent and less impact of the tissue movements during heart beating to the inner stent. The positive anchoring and force distribution may also be achieved by the particular radial design of the fixation means and their connection arches. In one aspect the connection of the connecting arches with the outer stent is advantageous because the two parts are only connected to each other in a defined and restricted area and optionally by the braid or cells of the outer stent being interwoven with the connecting arches at defined areas and not over the entire circumferential area of the outer stent. Also the fact that the inner and outer stent are not sutured together and are only connected at a restricted area supports uncoupling of the inner stent and supports an advantageous functionality of the valve placed within the inner stent. Thus an advantageous decoupling of the anchoring function and sealing function can be achieved.

In another advantageous aspect the stent and prosthesis according to the disclosure provides for a sealing by way of the outer stent and the related sealing materials attached thereto which serves the primary purpose of sealing and avoiding paravalvular leakage. The special design of the prosthesis advantageously decouples the outer stent from the basic anchoring functionality provided by the anchoring means and which is essentially decoupled from the outer stent. Thus the outer stent serves essentially for sealing and the sealing functionality is decoupled from the anchoring functionality in turn may result in an improved sealing characteristics by a better alignment of the outer sealing stent because it serves less or essentially not an anchoring function.

The advantageous sealing characteristics of the outer stent and advantageous alignment with the surrounding tissue of the target site may be further achieved not only by the decoupling but also by the use of a braided outer stent or relatively small dimensions of the cells of the laser cut outer stent which advantageously has a reduced stiffness as compared to known laser cut stents.

A more specific description of an embodiment according to the disclosure is a stent comprising an inner stent (2) and an outer stent (1) wherein the inner stent (2) comprises or is connected with at least one fixation means (18).

The number of fixation means (18) is chosen according to the other design features of the stent and also the replacement heart valve prosthesis. The inner stent (2) comprises or can be connected with 2, 3, 4, 5, 6, 7, 8, 9, 10, 20, 36 or more fixation means (18).

A more specific description of aspects of the disclosure is a replacement heart valve prosthesis, a method of implanting such a prosthesis and a method of positioning such a prosthesis.

In particular another aspect the problem underlying the application is solved by a replacement heart valve prosthesis comprising an inner stent and an outer stent as describe above and at least one sealing means in the inner and/or outer stent and a valve connected to the inner stent, wherein the valve has optionally 2 or 3 leaflets.

In another aspect the problem underlying the application is solved by a method for implanting a replacement heart valve prosthesis as describe above using a catheter based delivery system.

In another aspect the problem underlying the application is solved by a method for positioning a replacement heart valve prosthesis in a native tricuspid or mitral heart valve wherein the prosthesis is delivered by a catheter based delivery system to the target site (e.g. the native mitral or tricuspid annulus) wherein the target site is the annulus, the prosthesis get released from the delivery system into the right or left heart chamber, wherein area 11 including the anchoring loops (6) is essentially in the ventricle, area 10 is essentially in the area of the annulus and section 9 is essentially in the atrium positioned.

In particular, one solution according to the disclosure is in further detail a stent as disclosed herein wherein the at least one fixation means (18) is composed of or comprises an anchoring loop (6), an anchoring arm (7), a connecting arch (12) and an inner stent anchor (13), preferably a connecting means (4) connects the inner stent anchor (13) of the fixation means (18) with the inner stent (2).

A stent according to the disclosure can have various design which achieve the object. In one embodiment it is a stent wherein the inner stent (2) or/and outer stent (1) can be composed of multiple parts, or wherein the inner stent (2) or/and outer stent (1), and optionally the fixation means (18), are one integral part, e.g. laser cut from one nitinol tube, or are connected to each other.

In one embodiment the stent according to the disclosure is a stent wherein the fixation means (18) and the inner stent (2) are connected to each other by way of connecting means (4) at the atrial end or atrial area.

In a stent according to the disclosure the inner stent (2) exhibits a relatively high stiffness and the outer stent (1) exhibits a relatively low stiffness and has a relatively high flexibility in order to adapt and align with the target endogenous tissue. Thus it has been shown to be advantageous if the outer stent (1) has a higher flexibility compared to the inner stent (2). Thus the inner stent (2) has a higher stiffness as compared to the outer stent (1). Accordingly the skilled person will appreciate that he will chose appropriate materials which materials may be stainless steel, nitinol, plastics, composite materials or other known materials. In one embodiment according to the disclosure the inner stent (2) is a laser cut stent and the outer stent (1) is a laser cut stent or a braided stent, and wherein the inner stent (2) and/or outer laser cut stent (1) comprises 6 to 48 cells circumferentially and 1 to 108 cells longitudinally or the outer braided stent (1) comprises or is composed of 6 to 72 wire meshes circumferentially and 1 to 180 wire meshes longitudinally.

A stent according to the disclosure in one embodiment is composed of or comprises various structural and functional parts which may be made from various parts which are connected with each other with appropriate means. In a stent according to the disclosure the inner stent (2) and outer stent (1) are directly connected to each other by one or more sutures, a connecting mechanism, welding, riveting or/and sleeves, or the inner stent (2) and outer stent (1) are connected to each other by way of the connecting means (4).

Usually the inner stent (2) and the outer stent (1) are not directly connected but are linked by way of the fixation means (18). A stent according to the disclosure in one embodiment is a stent wherein at least one fixation means (18) is pushed or pulled through at least one cell of the outer stent (1), or at least one fixation means (18) is interwoven with the outer stent (1), or/and connected with sutures, one or more connecting means (4), by welding, riveting or/and one or more sleeves.

The stents according to the disclosure will be connected as it is useful in view of the entire design and functionality. A stent according to the disclosure in one embodiment is a stent wherein the inner stent (2) and outer stent (1) are connected to each other in the atrial area or at the atrial end.

In a stent according to the disclosure the fixation means (18) can be connected or positioned in the ventricular area or at the ventricular end or in the atrial area or at the atrial end of the inner stent (2).

It has been shown to be useful if the stent comprises means for an easier loading onto a catheter. In a stent according to the disclosure the outer stent (1) can comprise at least two loading loops (3) or/and a groove, e.g. a V or U groove (5).

Stent according to the disclosure can be defined in three sections, e.g. an atrial area (9), an annulus area (10) and a ventricular area (11).

The dimensions of the stent will be adapted to each other and the dimensions of the target area and space. In a stent according to the disclosure in one embodiment can have dimensions wherein the atrial area (9) has a diameter of 20 to 90 mm and a length of 2 to 30 mm, the annulus area (10) has a diameter of 10 to 80 mm and a length of 2 to 20 mm and the ventricular area (11) has a diameter of 20 to 90 mm and a length of 5 to 40 mm.

A stent according to the disclosure can be characterized in that the outer stent is connected with the fixation means (18) and the fixation means (18) is connected or forma a part of the inner stent. It can be advantageous if the area (12b) of the fixation means (18) is interwoven with the outer stent (1).

The skilled person will appreciate that the materials and the design and thickness or density of the materials of the stent will influence the overall prosthesis characteristics. In an embodiment wherein a wire braided stent is used as the outer stent (1), it can consist of or comprise from 1 to 108 wires.

The number of fixation means (18) can vary. In one embodiment of a stent according to the disclosure the number of connecting arms (7) and/or connecting arch (12) of the fixation means (18) is from 2 to 36.

The various dimensions of a stent according to the disclosure will be chosen as is useful and the anchoring loops (6) can exhibit an angle to the longitudinal axis of the stent of 0° to 45°, or/and have a length of 3 mm to 20 mm, or/and have a radius of 0.5 to 5mm.

In a stent according to the disclosure in one embodiment the stent may comprise an additional radial force wire (17), optionally increasing the radial force of the stent of at least 10% as compared to a stent without the radial force wire.

In a stent according to the disclosure the anchoring loop (6) can exhibit the following dimension (14), (15), (16) wherein the angle of (14) equals from 0° to 80°, (15) is 3 mm to 20 mm and (16) is 0,5 mm to 5 mm.

In addition to the features as described above the stent according to the disclosure can further comprise at least one radial force wire (17), or two, three or four radial force wires (17).

The anchoring loops support the fixation at the target site. In one embodiment a stent according to the disclosure comprises 1 to 54 anchoring loops (6).

The method as described above can be further characterized in that it relates to a method for implanting a replacement heart valve prosthesis using a catheter-based implantation and delivery system.

The catheter will be introduced as is appropriate with regards to the target native heart valve and the catheter system can be introduced trans-femoral, trans-atrial, trans-jugular, or trans-apical.

In another detail the disclosure relates to a method for positioning a replacement heart valve prosthesis in a native tricuspid or mitral heart valve wherein the prosthesis is delivered by a catheter based delivery system to the target site (e.g. the native mitral or tricuspid annulus), the prosthesis is released from the delivery system into the right or left heart chamber, wherein area 11 including the anchoring loops (6) is positioned essentially in the ventricle, area 10 is positioned essentially in the area of the annulus, and area 9 is positioned essentially in the atrium.

In said method the fixation means (18) are positioned with their anchoring loops (6) in the sub-annular area or/and ventricular area.

The prosthesis as disclosed herein can also be retrieved in case of sub-optimal positioning or other problems during the implantation procedure.

### Examples

The following examples serve to illustrate various embodiments of the disclosure. They are not meant to be interpreted as restrictive in any way.

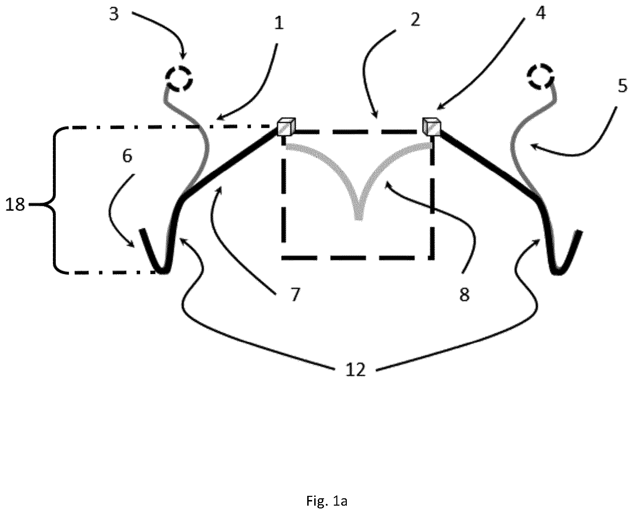

In Fig. 1a a two-part stent according to the disclosure is described comprising inner and outer stent (2, 1) wherein the inner stent (2) is connected by connecting means (4) with fixation means (18) comprising connecting arms (7), connecting arches (12) and anchoring loops (6). The connecting means (4) may exhibit a special design useful in medical devices and useful for the purpose and environment, e.g. it can be a clip mechanism or type of a screw. The connection can also be achieved by a welding process or other useful techniques to combine two parts known in the art. Inside the inner stent (2) the valve leaflets (8) are schematically indicated. The position of the valve leaflets (8) - two or three - may be adapted to the particular replacement valve type, e.g. tricuspid or mitral. Also the position within the inner stent (2) will vary depending on the replacement valve type and the particular size of the replacement valve type. Thus the valve leaflets (8) can be positioned e.g. closer to the connection of fixation means (18) and connecting means (4) that means closer to the upper part of the inner stent (2)-. It may be aligned with the annulus of the target site or further towards to the lower part of the inner stent (2) . In this embodiment according to the disclosure at the end areas of the outer stent (1) schematically loading loops (3) are shown. A prosthesis according to the disclosure may contain at least two, or three loading loops (3) but also e.g. five, seven, or nine or more. The advantage of two, three or five loading loops (3) is that in this way less space is needed when loaded onto the catheter system which advantageously reduced the overall diameter of the catheter system for delivery. Nevertheless with three loading loops a uniform pull can be achieved when loading the prosthesis onto the catheter. It may be useful to use more than three or five or seven, eight, nine or more loading loops (3) when using a very soft outer stent (1) in order to guarantee a uniform force is applied to the prosthesis during the loading procedure. The inner stent (2) is e.g. cut from a nitinol tube and exhibits a uniform design of cells. The cell design can also vary and the density of the cell can be chosen with a variation in order to vary the stiffness of the inner stent (2). As depicted here it has a uniform diameter over its length and it will maintain essentially it diameter during heart beat at the target site. The advantage is thus that the function of the valve leaflets (8) in this way will be comparable to a native valve and the valve leaflets will coapt in an advantageous manner. The angle between the connecting arm (7) and the connecting arch (12) may vary depending on the overall size of the prosthesis and the radial force that shall be achieved. Each of the connecting arm (7) and the connecting arch (12) can have an essentially straight design or each of them can be bent. The two parts or sections (distal (12) and proximal (7)) of the fixation means (18) can also form one arch with no angle and the parts or sections can form one slightly bended arch over the whole length. The anchoring loop (6) will have a dimension which is useful in the context of the overall design and with regards to the target site. The anchoring loop (6) can reverse in its direction with regards to the connecting arch (12) or it can protrude in the same direction and in addition contain at its very end a tip which is rounded in order to prevent damages in the target tissue. The anchoring loop (6) can be designed to be essentially parallel to the connecting arch (12) or form a certain angle with the connecting arch (12) of e.g. 10 ° to 45 °. The outer stent (1) will have a design that is useful for an optimized alignment with the target site and in order to align as good as possible with the target endogenous tissue. It may exhibit a groove, e.g. a U or V groove. Said groove will align with the annulus area and support a correct positioning and it will in addition be useful for fixation of the prosthesis at the target site. The groove will be positioned in relation to the other parts of the prosthesis, i.e. fixation means (18) and inner stent (2), in order to achieve an advantageous positioning at the target site and to avoid interference with a correct functioning of the replacement heart valve prosthesis. It will also serve to support a good compliance of the prosthesis with the heart and the overall heart function.

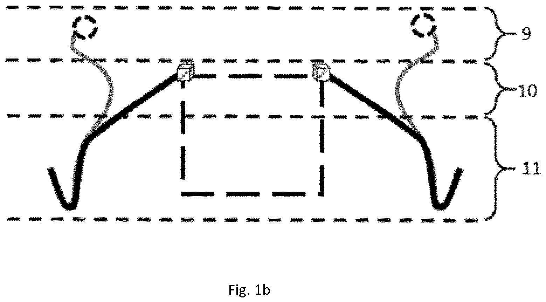

Fig. 1b shall illustrate an example of a replacement heart valve prosthesis according to the disclosure and its various areas, i.e. areas (9), (10), (11). Area (9) is the atrium area, area (10) is the annulus area and area (11) is the ventricular area. The fixation means (18 - the reference number is not shown) can extend over the distal end of the inner stent and also the outer stent can extend over the distal end of the inner stent. It will be appreciated that the longitudinal dimensions of the fixation means and the outer stent will be fine tuned depending on the particular size requirements of the target site.

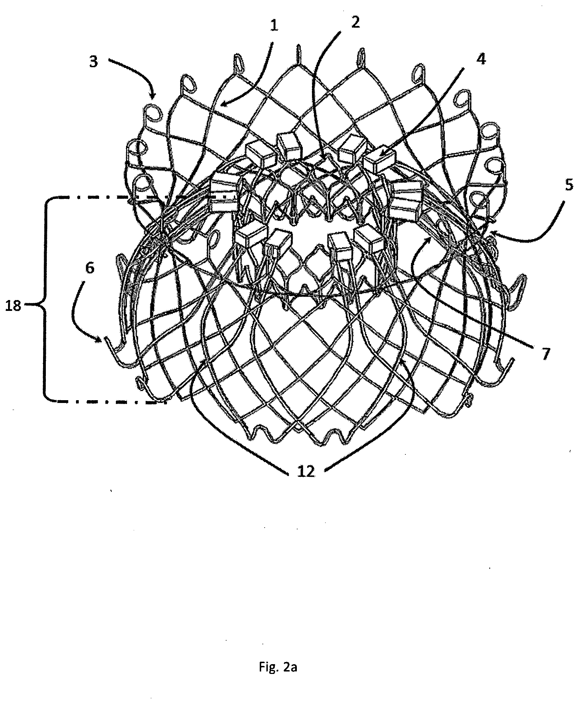

In Fig. 2a one can see outer stent (1) and inner stent (2) wherein the fixation means are connected with the inner stent (2) by way of 12 connecting means (4). The number of connecting means (4) can vary e.g. from 4 to 20. A fixation means has two connecting arms (7) wherein the two connecting arms (7) are essentially parallel and wherein the connecting arches (12) are spaced apart and which at their ends form a anchoring loop (6) which bends backward and in the opposite direction. Fixation means (18) are superimposed wherein the connecting arches (12) cross over each other. The inner stent (2) has two rows of cells but the number of rows can vary from 2 to 20.

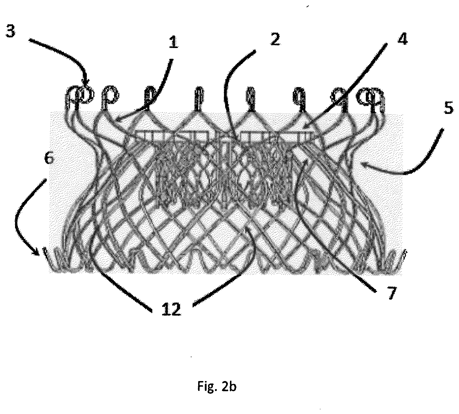

In Fig. 2b which is a side view of the replacement heart valve prosthesis one can see the groove (5) useful for an advantageous alignment of the prosthesis with its target site. Fixation loops (6) will align with the ventricular endogenous tissue.

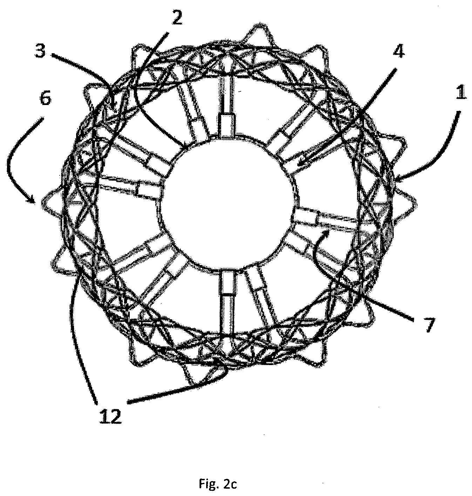

In Fig. 2c a top view is depicted and the orientation and spacing of the connecting means (4) is visible as well as the outward protrusion of the fixation loops (6). The outer stent (1) is connected via the fixation means (18) composed of connecting arms (7) and connecting arches (12) and fixation loops (6) with the inner stent (2). The outer stent (1) is not directly connected with inner stent (2). The outer stent serves for an advantageous alignment of the prosthesis with the target endogenous tissue and to mechanically isolate the outer stent (1) from the inner stent (2)supported by the fixation means (18). Thus advantageously the functionality of the replacement heart valve connected with the inner stent (2) is essentially not impaired during the heart contractions and movement of the endogenous tissue.

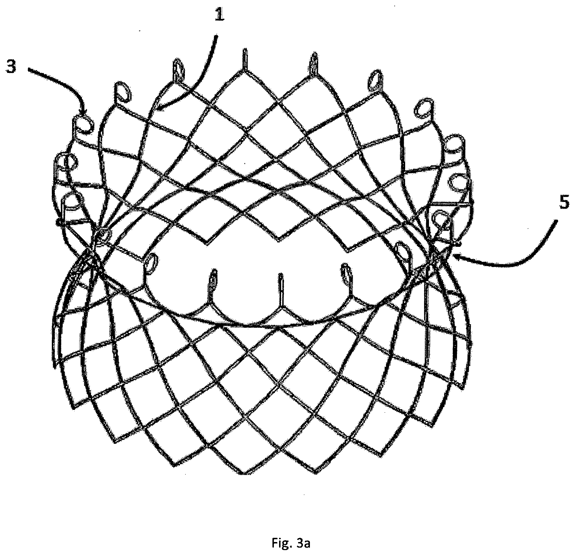

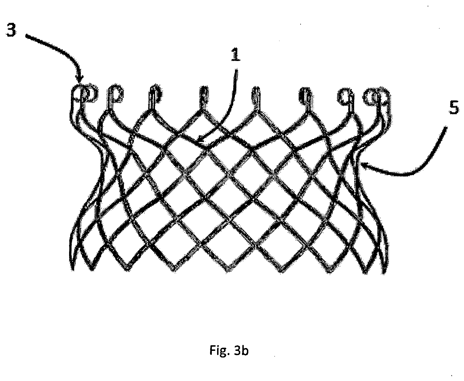

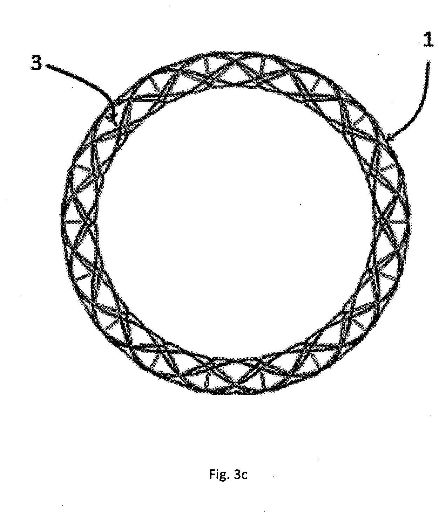

The Fig. 3a, 3b, 3c are variations of an outer stent (1) according to the disclosure viewed from different angles. The loading loops (3) have essentially an angle of 90 ° to the longitudinal direction of the prosthesis. Said loading loops (3) will align within the catheter during the loading procedure and tilt sideways in order to be stored during the loading onto the catheter.

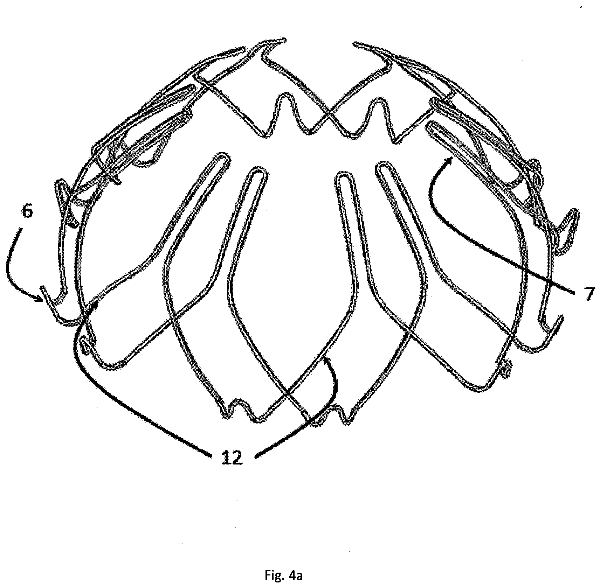

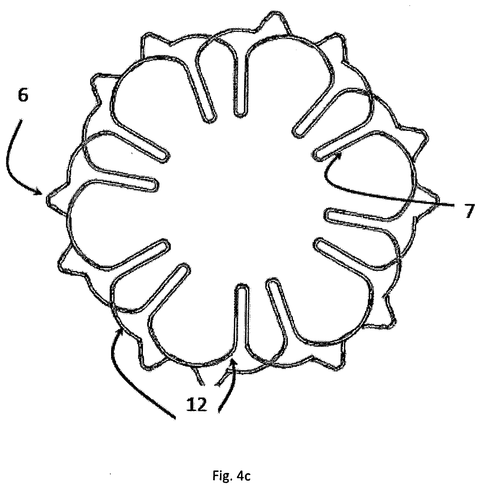

Fig. 4a to 7 show different embodiments and view directions of fixation means (18) according to the disclosure wherein the special design and orientation of the connecting arms (7), connecting arches (12) and anchoring loops (6) are shown. In particular in Fig. 4c the parallel orientation of the connecting arms (7) is obvious. Such a parallel orientation supports a certain stiffness in said area whereby the loops of the connecting arches (12) are useful for an advantageous alignment of said area with the target endogenous tissue. The orientation of the fixation loops (6) in the counter-direction is useful for a fixation in the target endogenous tissue.

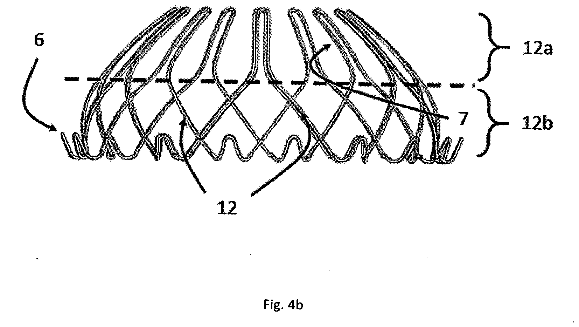

Fig. 4 a - c: shows another variation of the embodiments according to the disclosure. In order to increase the radial force in the ventricular area several connecting means (18) can be chosen in the stent design; one can e.g. chose 2 - 35 connecting means (18) which it is possible to adapt the radial force as desired by variation of the number of connecting means (18), e.g. 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35. In this manner the radial force may be fine tuned as the need will be and in connection with the other features of the stent requirements. Thus the radial force can be easily varied over a range depending on the number of connecting means (18).

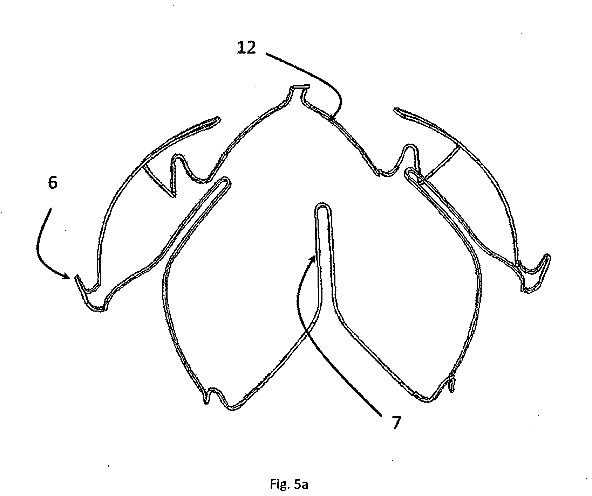

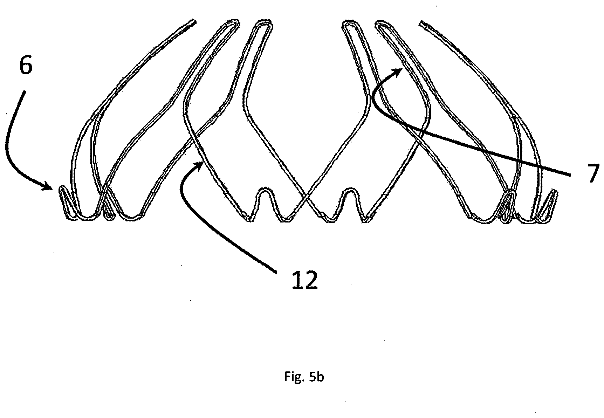

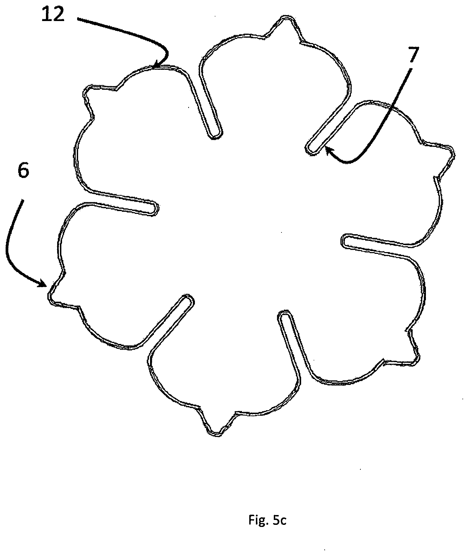

Fig. 5 a - c: shows another variation of the embodiments according to the disclosure. IT is possible to use solelyone connecting means (18) if the radial force of the outer stent (1) shall not be increased significantly in the ventricular area, however the number of anchoring loops (6) and connecting arms (7) shall not be reduced significantly. One can e.g. the angle of the mesh design reduce close to the anchoring loops (6); in this manner one can obtain a design wherein one connecting means (18) without crossing in this area.

In Fig. 5c the connecting arches (12) do not cross each other in contrast to the embodiment depicted in Fig. 4c.

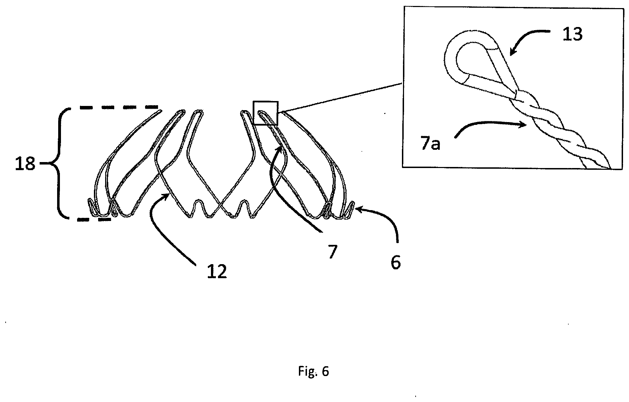

In Fig. 6 the fixation means (18) consisting of connecting arm (7), connecting arch (12) and anchoring loop (6) is shown and a special design of the connecting arm (7a) forming an inner stent anchor (13) serving for connection with the inner stent by way of connecting means (4). The connecting arm or arms (7) can be designed as one, two or more parallel struts or they can be twisted which will increase the stability or stiffness.

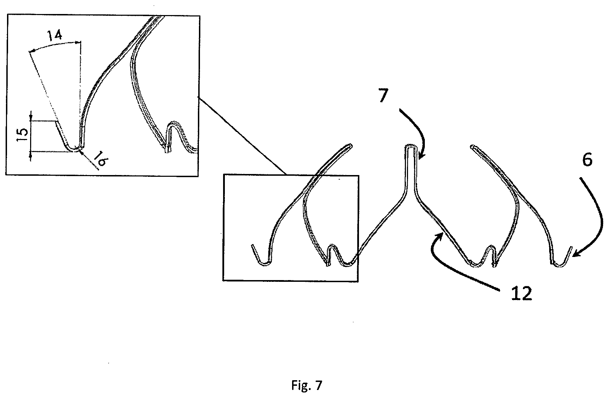

The anchoring loop (6) can vary in its design in various aspects as is shown in Fig. 7. One may e.g. introduce variations in the angle of the anchoring loop (14), the radius of the anchoring loop (16) and length of the anchoring loop (15). An angle of the anchoring loop (14) of 80° to 10° in relation to the connecting arch (12) can be advantageous for exhibiting a positive radial force in the area where the anchoring arch (6) will contact the native tissue and to supporting an improved fixation. An angle (6) of 15° to 25° can be advantageous. A variation of the length of the anchoring loop (6) of 5 to 15mm and/or a radius (16) of 1 to 4 mm can also be advantageous.

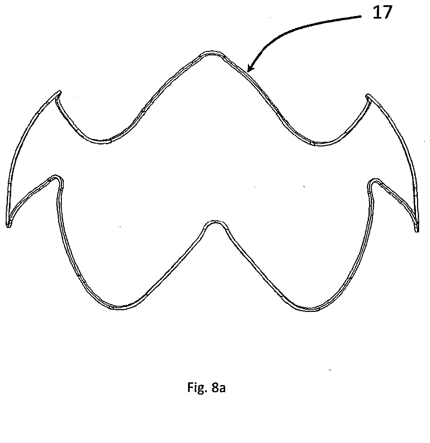

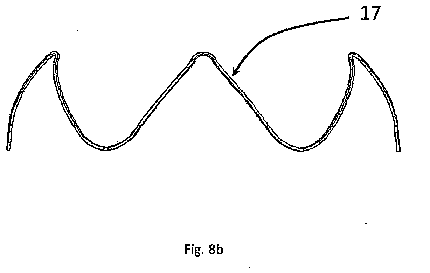

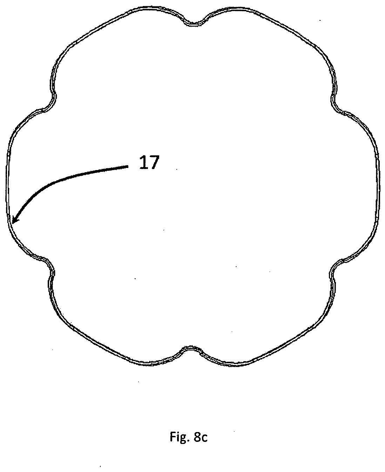

Fig. 8a, 8b, 8c show different embodiments of a radial force wire (17) according to the disclosure. A radial force wire (17) may be added in order to fine tune the radial force in a certain area of the prosthesis. Advantageously the radial force wire (17) is attached to the outer stent (1) in area (10) or (11) for an improved fixation of the prosthesis at the target site.

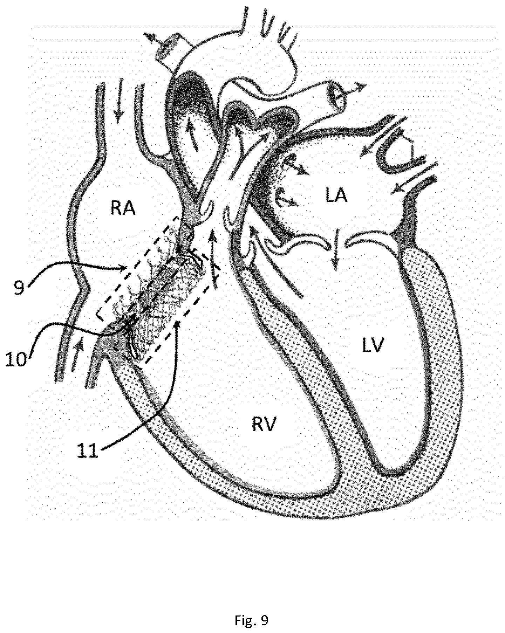

Fig. 9 illustrates the different areas (9), (10), (11) of a replacement heart valve prosthesis according to the disclosure implanted into a native tricuspid heart valve. The different areas (9), (10), (11) can be chosen to exhibit a certain length to each other for an advantageous design of the replacement heart valve prosthesis. Advantageous ratios of these areas or lengths are e.g. atrial area (9) has a diameter of 15 to 95 mm and a length of 1 to 35 mm, the annulus area (10) has a diameter of 8 to 85 mm and a length of 2 to 25 mm and the ventricular area (11) has a diameter of 18 to 95 mm and a length of 5 to 45 mm. The ratio of area (9) and/or area (11) to area (10) is 2:1 to 4:1. The ratio of the diameter of inner stent (2) to outer stent (1) can be chosen to be 1:1.4 to 1:3.5.

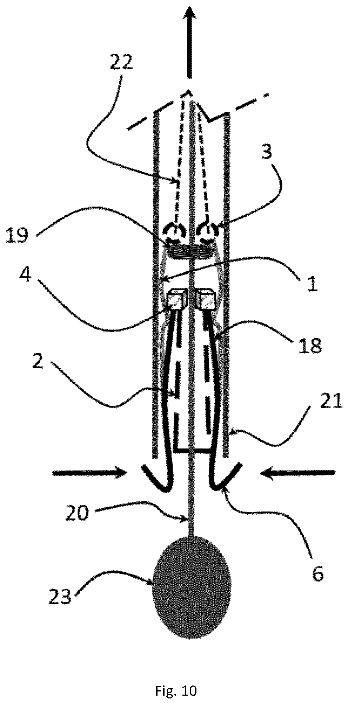

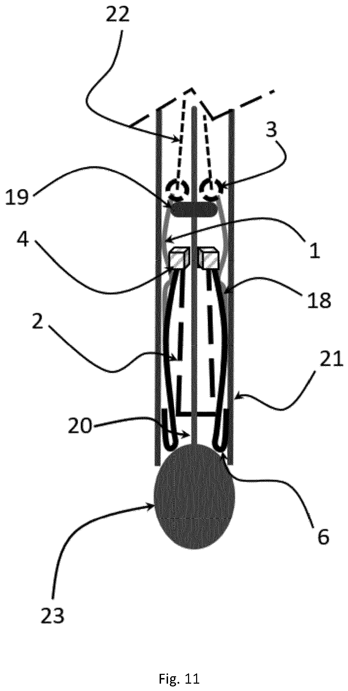

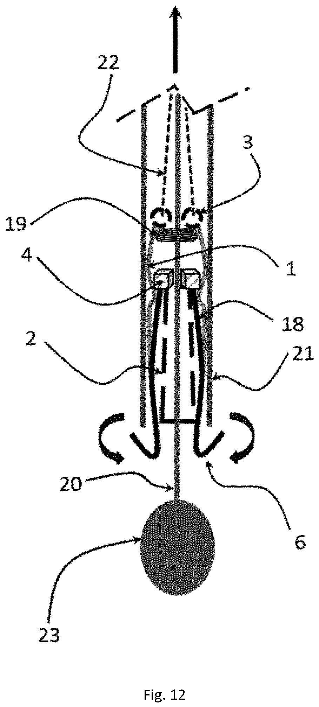

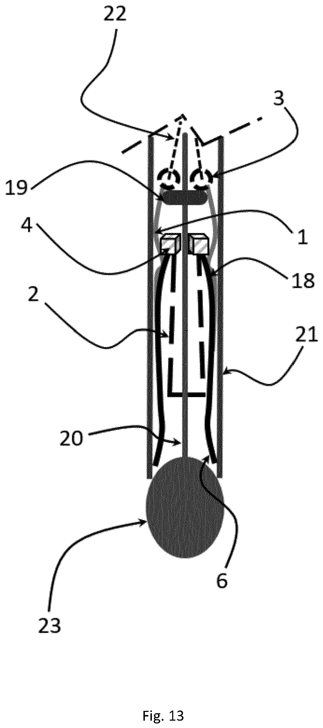

A loading procedure for a replacement heart valve prosthesis according to the disclosure is illustrated in Fig. 10 to 13 wherein the anchoring loops (6) can be folded and in said manner they can be loaded onto the catheter (Fig. 10, 11). Or alternatively the anchoring loops (6) can be stretched and unfolded in order to reduce the diameter of catheter and prosthesis (Fig. 12, 13). In both cases the prosthesis is loaded onto a catheter delivery system into a delivery capsule. The prosthesis can be fully covered and only partially covered by the outer catheter sheath. In Fig. 10 one can see that the anchoring lops (6) are pressed inwardly and covered by delivery system outer shaft (21). Delivery system tip (23) is pulled back by delivery system inner shaft (20) (Fig. 10, 11). In Fig. 12 and 13 it is shown that the anchoring loops (6) are folded and stretched longitudinally and delivery system tip (23) is pulled back by delivery system inner shaft (20).

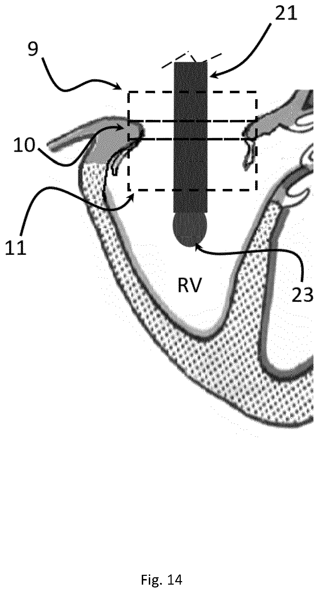

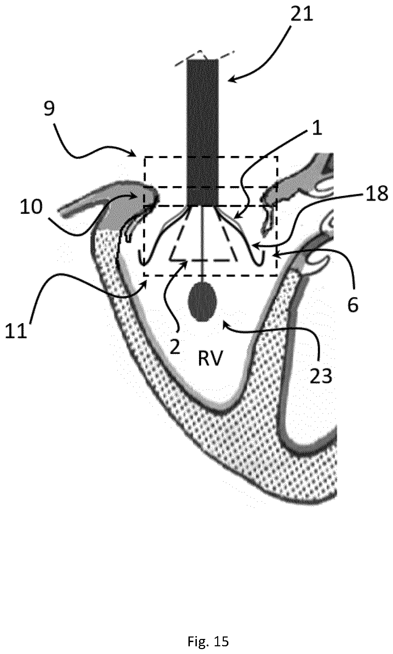

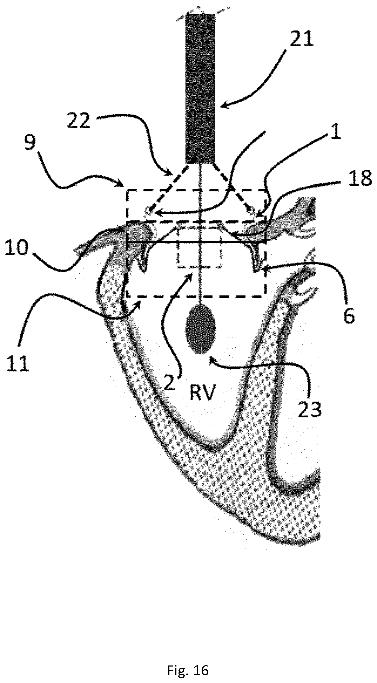

Fig. 14 to 16 is a sequence illustrating the deployment of a replacement heart valve prosthesis according to the disclosure at a target site in a patient heart (e.g. tricuspid). Fig. 14 shows alignment of the replacement heart valve prosthesis (still within the catheter capsule) with its areas (9), (10), (11) in the atrial, annulus and ventricular area of the native valve/heart and thus aligns correctly for an advantageous functionality. In Fig. 15 the catheter tip is pushed distally (seen from the operator of the catheter) and the prosthesis partly is released from the catheter wherein the fixation means (18) and inner stent (2) is visible in the ventricular area of the heart. In a next step illustrated in Fig. 16 the prosthesis is entirely released but still in connection with the catheter via retrievability connection (22). Thus the prosthesis can still be repositioned and retrieved by the catheter in case of complications. In Fig. 16 the prosthesis has correctly aligned with the target site and the native tricuspid valve area so that a correct functioning is achieved. Such an alignment is supported by groves (5) which direct the groove to the annulus and thus form part of the alignment function of the prosthesis according to the disclosure. The fixation means (18) does not only support fixation and a secured positioning but may also be useful to push back the native valve leaflets radially. After complete release and detachment of the retrievability connection (22) the catheter is pulled back and taken out of the patient's body and all further necessary actions are taken to finalize the procedure.

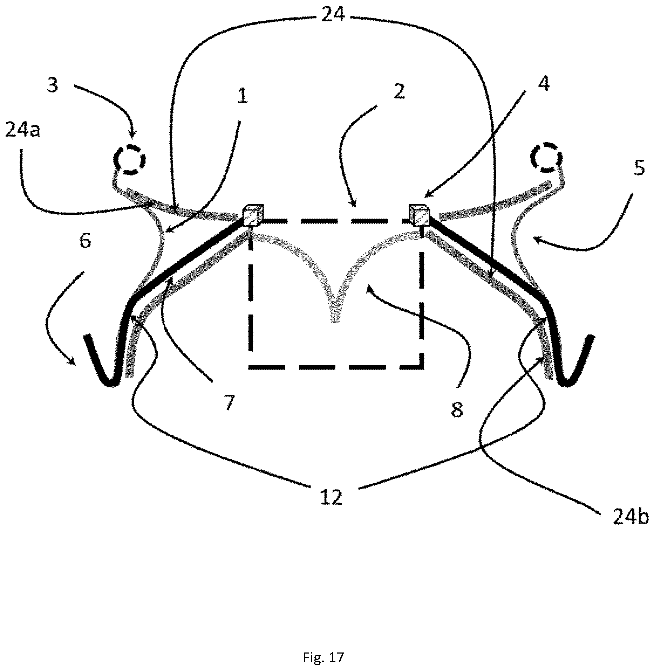

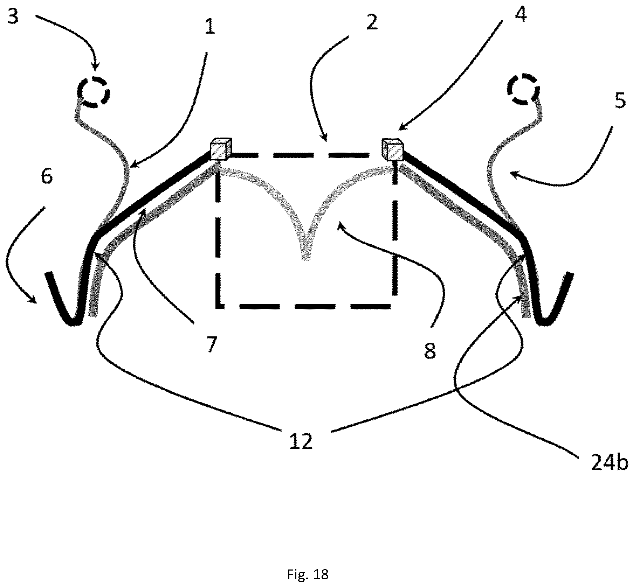

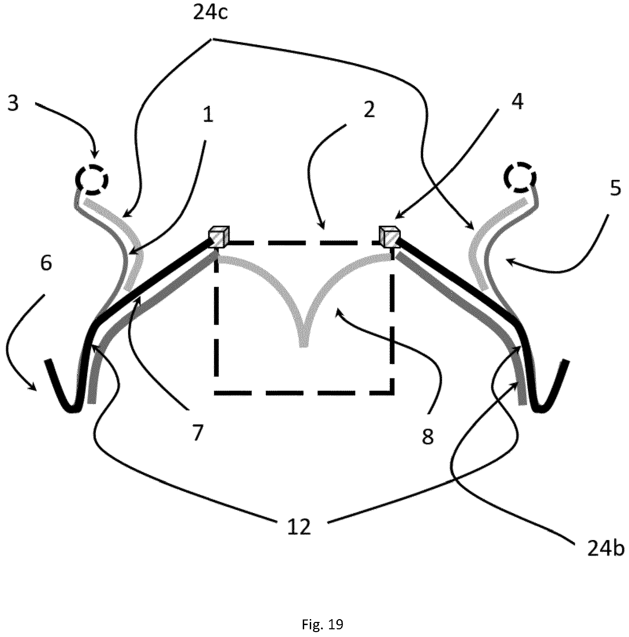

Fig. 17 to 19 are examples of sealing means (24, 24a, 24b, 24c) in a replacement heart valve prosthesis according to the disclosure. The sealing means can be combined depending on the particular prosthesis design and possible combination are e.g. atrial sealing (24a) and ventricular sealing (24b) (Fig. 17), solely ventricular sealing (24b) (Fig. 18), or a combination of annulus sealing (24c) and ventricular sealing (24b) (Fig. 19).

### Reference Number List

1 - outer stent (wire braided or laser cut)
2 - inner stent (laser cut)
3 - loading loops
4 - connecting means
5 - V or U groove
6 - anchoring loop
7 - connecting arm
7a - twisted connecting arm
8 - valve leaflet
9 - atrial (proximal) area
10 - annulus (middle) area
11 - ventricular (distal) area
12 - connecting arch
12a - atrial (proximal) connecting arch
12b - ventricular (distal) connecting arch
13 - inner stent anchor
14 - angle of anchoring loop
15 - length of anchoring loop
16 - radius of anchoring loop
17 - radial force wire
18 - fixation means
19 - stentholder
20 - delivery system inner shaft
21 - delivery system outer shaft
22 - retrievability connection
23 - delivery system tip
24 - sealing means
24a - atrial sealing
24b - ventricular sealing
24c - annular sealing

## Claims

1. A stent comprising an inner stent (2) and an outer stent (1) wherein the inner stent (2) comprises or is connected with at least one fixation means (18) and the inner stent (2) and the outer stent (1) are not directly connected (PCT p. 20, 1^{st} para) and the inner stent (2) and the outer stent (1) are essentially mechanically isolated from each other and the number of connecting arms (7) and/or connecting arch (12) of the fixation means (18) is from 2 to 36 (original claim 15), and at least one fixation means (18) is connected with one or more connecting means (4) (original claim 7), and wherein the fixation means (18) and the inner stent (2) are connected to each other by way of connecting means (4) at the atrial end or atrial area.

2. A stent according to claim 1, wherein the inner stent (2) comprises or is connected with 2, 3, 4, 5, 6, 7, 8, 9, 10, 20, 36 or more fixation means (18).

3. Stent according to claim 1 or 2, wherein the at least one fixation means (18) is composed of or comprises an anchoring loop (6), an anchoring arm (7), a connecting arch (12) and an inner stent anchor (13), preferably a connecting means (4) connects the inner stent anchor (13) of the fixation means (18) with the inner stent (2), or/and wherein the inner stent (2) or/and outer stent (1) can be composed of multiple parts, or wherein the inner stent (2) or/and outer stent (1), and optionally the fixation means (18), are one integral part, e.g. laser cut from one nitinol tube, or are connected to each other, and/or wherein the inner stent (2) is a laser cut stent and the outer stent (1) is a laser cut stent or a braided stent, and wherein the inner stent (2) and/or outer laser cut stent (1) comprises 6 to 48 cells circumferentially and 1 to 108 cells longitudinally or the outer braided stent (1) comprises or is composed of 6 to 72 wire meshes circumferentially and 1 to 180 wire meshes longitudinally.

4. Stent according to any of the preceding claims wherein the outer stent (1) comprises at least two loading loops (3) or/and a groove, e.g. a V or U groove (5).

5. Stent according to any of the preceding claims wherein the stent can be defined in three sections, e.g. an atrial area (9), an annulus area (10) and a ventricular area (11).

6. Stent according to claim 5 wherein the atrial area (9) has a diameter of 20 to 90 mm and a length of 2 to 30 mm, the annulus area (10) has a diameter of 10 to 80 mm and a length of 2 to 20 mm and the ventricular area (11) has a diameter of 20 to 90 mm and a length of 5 to 40 mm.

7. Stent according to claim 5 or 6 wherein the area (12b) of the fixation means (18) is interwoven with the outer stent (1).

8. Stent according to any of the preceding claims wherein the wire braided outer stent (1) consists of or comprises from 1 to 108 wires.

9. Stent according to any of the preceding claims wherein the anchoring loops (6) exhibit an angle to the longitudinal axis of the stent of 0° to 45°, or/and have a length of 3 mm to 20 mm, or/and have a radius of 0.5 to 5mm.

10. Stent according to any of the preceding claims wherein the stent comprises an additional radial force wire (17), optionally increasing the radial force of the stent of at least 10 % as compared to a stent without the radial force wire, and/or
wherein the anchoring loop (6) exhibits the following dimension (14), (15), (16) wherein the angle of (14) equals 0° bis 80°, (15) is 3 mm to 20 mm and (16) is 0,5 mm bis 5 mm, or/and
further comprising at least one radial force wire (17), or/and
wherein the stent comprises 1 to 54 anchoring loops (6).

11. A replacement heart valve prosthesis comprising a stent according to any of claims 1 to 10 and wherein the inner stent (2) and/or outer stent (1) comprise at least one sealing means (24a, 24b, 24c) and a valve connected to the inner stent (2), wherein the valve has optionally 2 or 3 valve leaflets (8).

## Patentansprüche

1. Stent, umfassend einen inneren Stent (2) und einen äußeren Stent (1), wobei der innere Stent (2) mindestens ein Fixierungsmittel (18) umfasst oder mit diesem verbunden ist und der innere Stent (2) und der äußere Stent (1) nicht direkt miteinander verbunden sind und der innere Stent (2) und der äußere Stent (1) im Wesentlichen mechanisch voneinander isoliert sind und die Anzahl der Verbindungsarme (7) und/oder des Verbindungsbogens (12) des Fixierungsmittels (18) 2 bis 36 beträgt, und mindestens ein Fixierungsmittel (18) mit einem oder mehreren Verbindungsmitteln (4) verbunden ist, und wobei das Fixierungsmittel (18) und der innere Stent (2) am Vorhofende oder im Vorhofbereich über Verbindungsmittel (4) miteinander verbunden sind.

2. Stent nach Anspruch 1, wobei der innere Stent (2) 2, 3, 4, 5, 6, 7, 8, 9, 10, 20, 36 oder mehr Fixierungsmittel (18) umfasst oder mit diesen verbunden ist.

3. Stent nach Anspruch 1 oder 2, wobei das mindestens ein Fixierungsmittel (18) aus einer Verankerungsschlaufe (6), einem Verankerungsarm (7), einem Verbindungsbogen (12) und einem inneren Stentanker (13) besteht oder diese umfasst, vorzugsweise ein Verbindungsmittel (4) den inneren Stentanker (13) des Fixierungsmittels (18) mit dem inneren Stent (2) verbindet, oder/und wobei der innere Stent (2) oder/und äußere Stent (1) aus mehreren Teilen zusammengesetzt sein kann/können, oder wobei der innere Stent (2) oder/und äußere Stent (1), und gegebenenfalls die Befestigungsmittel (18) ein einstückiges Teil sind, z. B. aus einem Nitinolrohr lasergeschnitten, oder miteinander verbunden sind, und/oder wobei der innere Stent (2) ein lasergeschnittener Stent ist und der äußere Stent (1) ein lasergeschnittener Stent oder ein geflochtener Stent ist, und wobei der innere Stent (2) und/oder der äußere lasergeschnittene Stent (1) 6 bis 48 Zellen in Umfangsrichtung und 1 bis 108 Zellen in Längsrichtung umfasst oder der äußere geflochtene Stent (1) 6 bis 72 Drahtmaschen in Umfangsrichtung und 1 bis 180 Drahtmaschen in Längsrichtung umfasst oder daraus besteht.

4. Stent nach einem der vorhergehenden Ansprüche, wobei der äußere Stent (1) mindestens zwei Ladeschlaufen (3) oder/und eine Rille, z.B. eine V- oder U-Rille (5), aufweist.

5. Stent nach einem der vorhergehenden Ansprüche, wobei der Stent in drei Abschnitte unterteilt werden kann, z.B. in einen Vorhofbereich (9), einen Ringbereich (10) und einen Ventrikelbereich (11).

6. Stent nach Anspruch 5, wobei der Vorhofbereich (9) einen Durchmesser von 20 bis 90 mm und eine Länge von 2 bis 30 mm hat, der Ringbereich (10) einen Durchmesser von 10 bis 80 mm und eine Länge von 2 bis 20 mm hat und der Ventrikelbereich (11) einen Durchmesser von 20 bis 90 mm und eine Länge von 5 bis 40 mm hat.

7. Stent nach Anspruch 5 oder 6, wobei der Bereich (12b) des Fixierungsmittels (18) mit dem äußeren Stent (1) verwoben ist.

8. Stent nach einem der vorhergehenden Ansprüche, wobei der drahtgeflochtene äußere Stent (1) aus 1 bis 108 Drähten besteht oder diese umfasst.

9. Stent nach einem der vorhergehenden Ansprüche, wobei die Verankerungsschlaufen (6) einen Winkel zur Längsachse des Stents von 0° bis 45° aufweisen oder/und eine Länge von 3 mm bis 20 mm haben oder/und einen Radius von 0,5 bis 5 mm haben.

10. Stent nach einem der vorhergehenden Ansprüche, wobei der Stent einen zusätzlichen Radialkraftdraht (17) umfasst, der gegebenenfalls die Radialkraft des Stents um mindestens 10 % im Vergleich zu einem Stent ohne den Radialkraftdraht erhöht, und/oder
wobei die Verankerungsschlaufe (6) die folgenden Abmessungen (14), (15), (16) aufweist, wobei der Winkel von (14) 0° bis 80°, (15) 3 mm bis 20 mm und (16) 0,5 mm bis 5 mm beträgt, oder/und
ferner mindestens einen Radialkraftdraht (17) umfasst, oder/und
wobei der Stent 1 bis 54 Verankerungsschlaufen (6) aufweist.

11. Herzklappenersatzprothese mit einem Stent nach einem der Ansprüche 1 bis 10, wobei der innere Stent (2) und/oder der äußere Stent (1) mindestens ein Dichtungsmittel (24a, 24b, 24c) und eine mit dem inneren Stent (2) verbundene Klappe aufweisen, wobei die Klappe gegebenenfalls 2 oder 3 Klappensegel (8) aufweist.

## Revendications

1. Endoprothèse, comprenant une endoprothèse interne (2) et une endoprothèse externe (1), dans laquelle
l'endoprothèse interne (2) comprend ou est reliée à au moins un moyen de fixation (18) et l'endoprothèse interne (2) et l'endoprothèse externe (1) ne sont pas directement reliées, et l'endoprothèse interne (2) et l'endoprothèse externe (1) sont essentiellement isolées mécaniquement l'une de l'autre, et le nombre de bras de connexion (7) et/ou d'arcs de connexion (12) du moyen de fixation (18) est compris entre 2 et 36, et au moins un moyen de fixation (18) est relié à un ou plusieurs moyens de connexion (4), et dans laquelle le moyen de fixation (18) et l'endoprothèse interne (2) sont reliés l'un à l'autre par le moyen de connexion (4) à l'extrémité auriculaire ou à la zone auriculaire.

2. Endoprothèse selon la revendication 1, dans laquelle l'endoprothèse interne (2) comprend ou est reliée à 2, 3, 4, 5, 6, 7, 8, 9, 10, 20, 36 ou plus de moyens de fixation (18).

3. Endoprothèse selon la revendication 1 ou 2, dans laquelle l'au moins un moyen de fixation (18) est composé de ou comprend une boucle d'ancrage (6), un bras d'ancrage (7), un arc de connexion (12) et une ancre d'endoprothèse interne (13), de préférence, un moyen de connexion (4) relie l'ancre d'endoprothèse interne (13) du moyen de fixation (18) à l'endoprothèse interne (2), ou/et dans laquelle l'endoprothèse interne (2) ou/et l'endoprothèse externe (1) peuvent être composées de plusieurs parties, ou dans lequel l'endoprothèse interne (2) ou/et l'endoprothèse externe (1), et éventuellement les moyens de fixation (18), sont une seule et même pièce, par exemple découpée au laser dans un tube de nitinol, ou sont reliés entre eux, et/ou
dans laquelle l'endoprothèse interne (2) est une endoprothèse découpée au laser et l'endoprothèse externe (1) est une endoprothèse découpée au laser ou une endoprothèse tressée, et dans laquelle l'endoprothèse interne (2) et/ou l'endoprothèse externe découpée au laser (1) comprend de 6 à 48 cellules dans la circonférence et de 1 à 108 cellules dans la longueur ou l'endoprothèse externe tressée (1) comprend ou est composé de 6 à 72 mailles métalliques dans la circonférence et de 1 à 180 mailles métalliques dans la longueur.

4. Endoprothèse selon l'une quelconque des revendications précédentes, dans laquelle l'endoprothèse externe (1) comprend au moins deux boucles de chargement (3) ou/et une rainure, par exemple une rainure en V ou en U (5).

5. Endoprothèse selon l'une quelconque des revendications précédentes, dans laquelle l'endoprothèse peut être défini en trois sections, par exemple une zone auriculaire (9), une zone annulaire (10) et une zone ventriculaire (11).

6. Endoprothèse selon la revendication 5, dans laquelle la zone auriculaire (9) a un diamètre de 20 à 90 mm et une longueur de 2 à 30 mm, la zone annulaire (10) a un diamètre de 10 à 80 mm et une longueur de 2 à 20 mm et la zone ventriculaire (11) a un diamètre de 20 à 90 mm et une longueur de 5 à 40 mm.

7. Endoprothèse selon la revendication 5 ou 6, dans laquelle la zone (12b) du moyen de fixation (18) est entrelacée avec l'endoprothèse externe (1).

8. Endoprothèse selon l'une quelconque des revendications précédentes, dans laquelle l'endoprothèse externe tressée (1) est constituée de ou comprend de 1 à 108 fils.

9. Endoprothèse selon l'une quelconque des revendications précédentes, dans laquelle les boucles d'ancrage (6) présentent un angle par rapport à l'axe longitudinal de l'endoprothèse de 0° à 45°, ou/et ont une longueur de 3 mm à 20 mm, ou/et ont un rayon de 0,5 à 5 mm.

10. Endoprothèse selon l'une quelconque des revendications précédentes, dans laquelle l'endoprothèse comprend un fil de force radiale supplémentaire (17), augmentant éventuellement la force radiale de l'endoprothèse d'au moins 10 % par rapport à une endoprothèse dépourvue de fil de force radiale, et/ou dans laquelle la boucle d'ancrage (6) présente les dimensions suivantes (14), (15), (16), l'angle de (14) étant compris entre 0° et 80°, celui de (15) entre 3 mm et 20 mm et celui de (16) entre 0,5 mm et 5 mm, ou/et comprenant en outre au moins un fil de force radial (17), ou/et l'endoprothèse comprenant de 1 à 54 boucles d'ancrage (6).

11. Prothèse de remplacement de valvule cardiaque comprenant une endoprothèse selon l'une quelconque des revendications 1 à 10 et dans laquelle l'endoprothèse interne (2) et/ou l'endoprothèse externe (1) comprennent au moins un moyen d'étanchéité (24a, 24b, 24c) et une valvule connectée à l'endoprothèse interne (2), la valvule ayant éventuellement 2 ou 3 feuillets de valvule (8).
